(19) Europäisches Patentamt
European Patent Office
Office européen des brevets

(11) Publication number: **0 043 472 B1**

(12) # EUROPEAN PATENT SPECIFICATION

(45) Date of publication of the patent specification: **20.07.83**

(51) Int. Cl.³: **C 07 F 9/10,** C 07 F 9/09, A 61 K 31/685

(21) Application number: **81104668.9**

(22) Date of filing: **17.06.81**

(54) **3-Hydrocarbylthio-2-acyloxypropyl 2-trimethylammonioethyl phosphates, process for producing the same and pharmaceutical preparations containing the same.**

(30) Priority: **08.07.80 DE 3025804**

(43) Date of publication of application: **13.01.82 Bulletin 82/2**

(45) Publication of the grant of the patent: **20.07.83 Bulletin 83/29**

(84) Designated Contracting States: **AT BE CH FR GB IT LI LU NL SE**

(56) References cited:
**GB-A-2 020 663**
**US-A-3 542 820**

**CHEMICAL ABSTRACTS, vol. 92, no. 9, March 3, 1980, page 257, abstract 71752g, Columbus, Ohio, USA, D. LOMBARDO et al.: "Studies on the substrate specificity of a carboxyl ester hydrolase from human pancreatic juice. I. Action of carboxyl esters, glycerides and phospholipids"**

(73) Proprietor: **A. Nattermann & Cie. GmbH, Nattermannallee 1, D-5000 Köln 30 (DE)**

(72) Inventor: **Betzing, Hans, Dr., Heidstock 7, D-5014 Kerpen-Horrem (DE)**
Inventor: **Wirtz-Peitz, Ferdinand, Dr., Am Angelsdorn 9, D-5024 Pulheim (DE)**
Inventor: **Prop, Gerrit, Dr., Mohnblumenweg 25, D-5024 Pulheim (DE)**

(74) Representative: **Redies, Bernd, Dr. rer. nat. et al, Redies, Redies, Türk & Gille Brucknerstrasse 20, D-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

3-Hydrocarbylthio-2-acyloxypropyl 2-trimethylammonioethyl phosphates, process for producing the same and pharmaceutical preparations containing the same.

The present invention is related to new 3-hydrocarbylthio-2-acyloxypropyl 2-trimethylammonioethyl phosphate having the general formula I

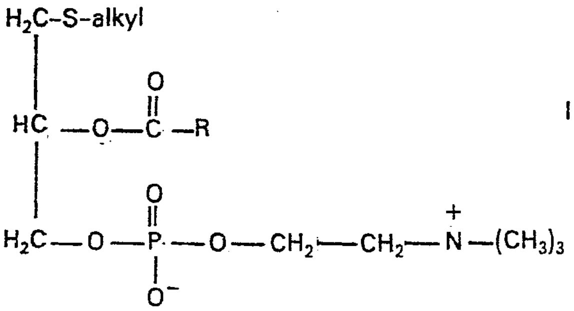

I

wherein alkyl is a saturated straight or branched acyclic hydrocarbon group with 6 to 20 carbon atoms or an unsaturated straight or branched acyclic hydrocarbon group with 6 to 20 carbon atoms containing one or two double bonds, and R represents methyl or ethyl, said new compounds having useful biological and pharmacological properties. The present invention is further related to a process for producing these compounds as well as to pharmaceutical preparations containing the same. The preferred compounds according to the present invention are those wherein alkyl represents a saturated straight or branched acyclic hydrocarbon group having from 12 to 20 carbon atoms or an unsaturated straight or branched acyclic hydrocarbon group having from 12 to 20 carbon atoms containing 1 or 2 double bonds. Since among such glycerides such compounds are of particular importance which are derivatives of natural fats, those compounds of formula I are most preferred wherein alkyl represents a saturated straight chain acyclic hydrocarbon group having an even number of carbon atoms. Such compounds are:

3-hexadecylthio-2-acetoxypropyl 2-trimethylammonioethyl phosphate;
3-hexadecylthio-2-propionyloxy 2-trimethylammonioethyl phosphate;
3-octadecylthio-2-acetoxypropyl 2-trimethylammonioethyl phosphate;
3-octadecylthio-2-propionyloxy 2-trimethylammonioethyl phosphate;
3-eicosylthio-2-acetoxypropyl 2-trimethylammonioethyl phosphate;
3-tetradecylthio-2-acetoxypropyl 2-trimethylammonioethyl phosphate;
3-tetradecylthio-2-propionyloxypropyl 2-trimethylammonioethyl phosphate;
3-dodecylthio-2-acetoxypropyl 2-trimethylammonioethyl phosphate;
3-dodecylthio-2-propionyloxypropyl 2-trimethylammonioethyl phosphate;

i.e. compounds of formula I wherein alkyl is a saturated straight chain acyclic hydrocarbon group having 12, 14, 16, 18 or 20 carbon atoms and the acyl group is an acetyl or propionyl group.

The compounds according to the present invention differ from the glycerol ether phospholipides, in particular 3-alkoxy-2-acyloxypropyl 2-trimethylammonioethyl phosphates in that the alkyl group in the 3-position of the propyl group is bound by a thioether linkage. The new compounds show interesting pharmacological properties such as a good antihypertensive activity. Therefor, the compounds according to the present invention are in particular used as active agent in pharmaceutical preparations for the treatment of increased blood pressure and of the thromboembolic diseases.

The new compounds according to the present invention are administered enterally or parenterally in dosages amounting to 0.1 to 100 mg., preferably 0.1 to 10 mg. Suitable galenic products are for instance tablets, capsules, solutions, suspensions or emulsions. Usual galenic auxiliary agents, cariers or lubricants or agents producing a retardent effect may be added in the preparations of pharmaceutical products. The compositions are prepared in the usual manner using methods of preparation.

The phospholipid compounds according to the present invention are produced by reacting 1-mercapto-2,3-propanediol with an alkyl-Hal, alkyl having the same meaning as in formula I and Hal representing a halogen atom, possibly in the presence of an alkaline condensation agent, blocking the primery hydroxy group in the 3-position of the resulting 1-alkylthio-2,3-propanediol with an appropriate protective group, esterifying the resulting protected product with a reactive derivate of a carboxylic acid of the formula R-CO-OH, R having the same meaning as in formula I, splitting off the protective group in the 3-position, esterifying the resulting 1-alkylthio-2-O-acyl-2.3-propanediol of the general formula V

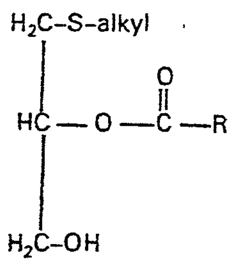

V

wherein alkyl and R have the same meaning as in formula I, with β-bromoethyl-phosphoric acid dichloride of the formula VI

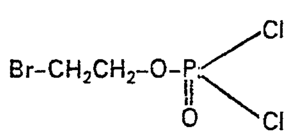

VI

and reacting the resulting 3-alkylthio-2-acyloxypropyl 2-bromoethyl hydrogen phosphate having the general formula VII

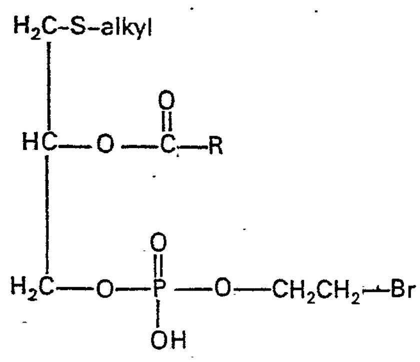

VII

wherein alkyl and R have the same meaning as in formula I, with trimethylamine and thereby converting it into the corresponding choline ester for formula I.

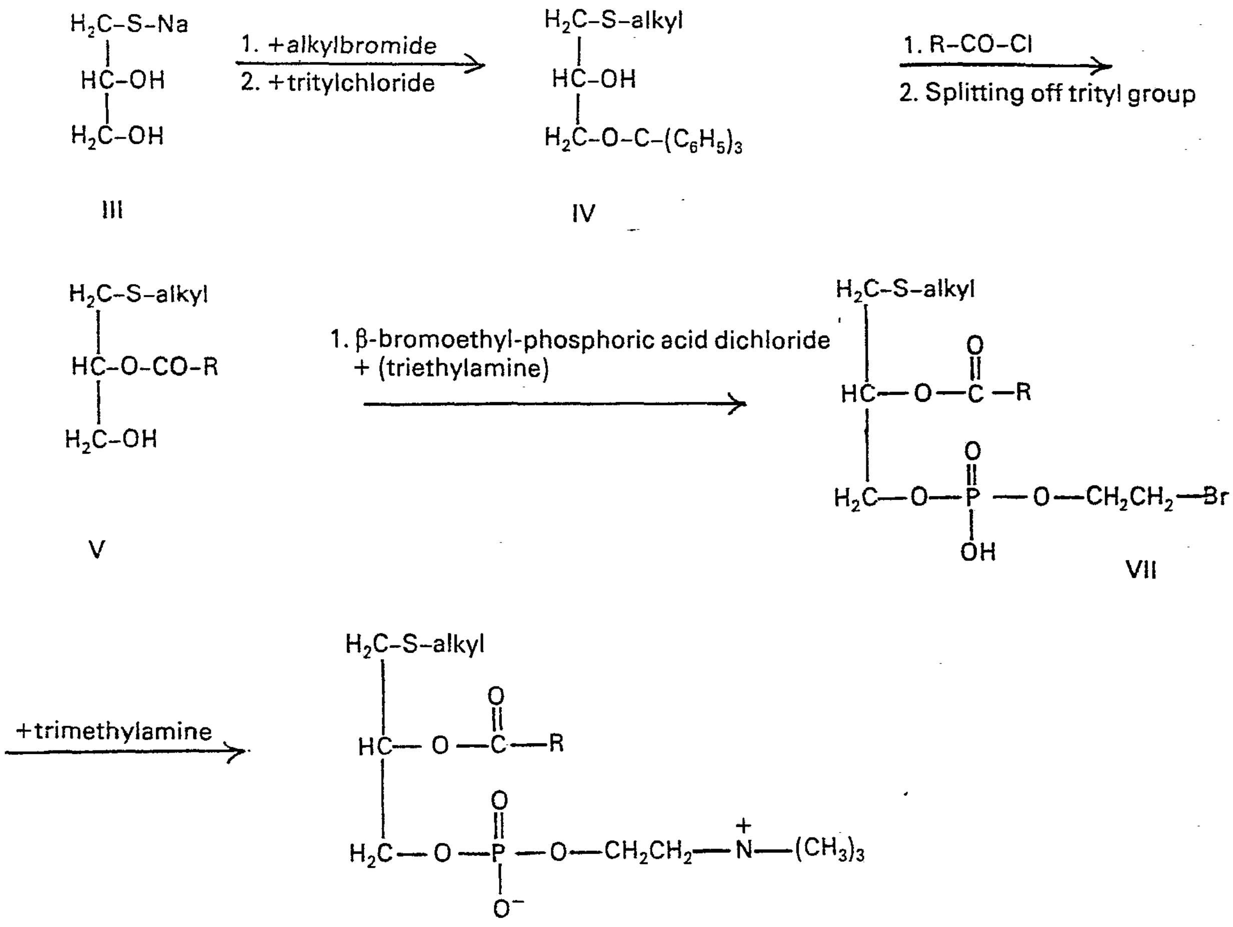


III IV V VII I

Reactive derivatives of the acid of the formula R-CO-OH suitable for esterification are in particular the acid halides such as acid chlorides or bromides, or acid anhydrides.

Suitable protective groups for blocking the primary hydroxy group in the 3-position of the 1-thioalkyl-2.3-propanediols are known to the expert (see for instance H. M. Verheij, Fundamentals of Lipid Chemistry, BI-Science Publications Division, Webster Groves, Missouri [USA], p. 225–249, in particular p. 234).

The reactions are carried out in inert organic solvents such as chloroform, tetrahydrofuran or toluence, preferably in the presence of a suitable acid binding agent such as in particular triethylamine, pyridine or quinoline as organic bases. Usually, the reaction components are reacted in equimolar amounts. In order to increase the yield of the reaction, one of the reaction components may be added in an excess. It is most desired to carry out all of the reactions with the exclusion of moisture, preferably in a temperature range from 10 to 60°C. One may however work at more elevated or lower temperatures in particular cases, i.e. the reaction is carried out in an overall temperature range of from 0 to 100°C.

The compounds according to the present application are amorphous semi-solid colourless

compounds having no defined melting points. The compounds may be characterized by thin layer chromatography, by determining the phosphorus content or by elementary analyses.

Example 1

a) 13 g. (0.1 mole) of the 1-sodium salt of 1-mercapto-2.3-propanediol in 200 cc. of methanol are stirred with 31 g. (0.1 mole) of 1-bromo-hexadecane for 10 to 15 hours at room temperature with the exclusion of moisture. The resulting precipitate is filtred off and washed with methanol several times. Thus, 30 g. of 1-hexadecylthio-2.3-propanediol (90% of the theoretical) are obtained.

b) 20 g. (64 mmole) of 1-hexadecylthio-2.3-propanediol are dissolved in 400 cc. of anhydrous tetrahydrofuran. After the addition of 10 g. of pyridine with 22.3 g. (80 mmole) of triphenylchloromethane, the reaction mixture is heated to 70°C. for about 30 hours. After cooling to room temperature, the precipitated pyridine hydrochloride is filtered off with suction and tetrahydrofuran is distilled off in a vacuum. The residue is dissolved in petroleum ether, the solution is cooled and the resulting precipitate is filtered off. Upon distilling off the petroleum ether, 30 g. of crude 1-hexadecylthio-3-O-trityl-2.3-propanediol (85% of the theoretical) are obtained.

c) The product according to b) above (52 mmole) is dissolved in 200 cc. of anhydrous chloroform. 8.0 cc. of triethylamine are added thereto and then 5.1 cc. of acetyl chloride are slowly added dropwise with cooling in an ice bath. Stirring of the reaction mixture is continued over night at room temperature. After termination of the reaction (DC-check) the chloroform solution is repeatedly washed with water, dried over sodium sulfate and the solvent is distilled off in a vacuum. The resulting 1-hexadecylthio-2-O-acetyl-3-O-trityl-2.3-propanediol shows an Rf-value of 0.73 using n-hexan/ether (85:15 v/v) as eluant.

25 g. (40 mmole) of this compound are refluxed for about 20 hours with 200 cc. of 75% aqueous acetic acid. The reaction mixture thereafter is poured into water which is extracted with diethyl ether. The resulting 1-hexadecylthio-2-O-acetyl-2.3-propanediol is purified chromatographically through a column of silicid acid.

Yield: 9.5 g. (66% of the theoretical), Rf-value: 0.71 eluant: chloroform/methanol (98:2 v/v).

d) 9 g. (25 mmole) of 1-hexadecylthio-2-O-acetyl-2.3-propanediol are dissolved in 20 cc. of absolute chloroform. A solution of 18.1 g. (75 mmole) of β-bromoethylphosphoric acid dichloride and 15.3 g. (150 mmole) anhydrous triethylamine in 40 cc. of anhydrous chloroform are added slowly dropwise thereto with stirring and cooling with ice. After the addition is terminated, stirring is continued at room temperature for 4 hours and at 37°C. for 15 hours. The reaction mixture thereafter is cooled to 0°C. and the resulting phosphoric acid ester chloride is hydrolyzed by the addition of 50 cc. of 0.1 N salt solution. Thereafter, the solution is stirred for 2 hours at 0°C. After the addition of 30 cc. of methanol the solution is acidified with HCl, the organic phase is separated and extracted once more with water and thereafter dried over MgSO. The solvent is distilled off and the residue is dried in a vacuum over $P_2O_5$. The yield of crude 3-hexadecylthio-2-acetoxypropyl-2-bromoethyl hydrogen phosphate amounts to 10.5 g. (75% of the theoretical), Rf-value: 0.55 with the eluant chloroform/methanol/water (65:25:4 v/v/v).

e) 10 g. (18 mmole) of the β-bromoethylphosphoric acid ester obtained according to d) are dissolved in 50 cc. of toluene without further purification. 15 cc of trimethylamine are added thereto at 0°C. and the reaction mixture is heated in a tightly closed autoclave for 15 hours and 55°C. The solvent then is distilled off in a vacuum, the residue is dissolved in 100 cc. of methanol, 6 g. of silver nitrate are added and the resulting reaction mixture is allowed to stand for 30 minutes at 50°C. with agitation from time to time. The silver salt is filtered off and the filtrate is evaporated in a vacuum. The residue is dried over $P_2O_5$ and is purified chromatographically through a column of silica gel.

Yield: 9.6 g (64% of the theoretical) of 3-hexadecylthio-2-acetoxypropyl-2-trimethylammonioethyl phosphate as a calourless, waxy substance. Rf-value: 0.28 with the eluant chloroform/methanol/water (65:25:4 v/v/v).

Elementary analyses: $C_{26}H_{54}O_6PNS$ (Mg. 539.31)

| | C | H | N | P | S |
|---|---|---|---|---|---|
| calculated: | 57.90% | 10.01 | 2.60 | 5.74 | 5.94 |
| found: | 57.69% | 10.11 | 2.45 | 5.52 | 5.82 |

Example 2

As described in example 1 the compounds of table 1 are produced:

| | Formula Mol. Weight | | C | H | N | P |
|---|---|---|---|---|---|---|
| 3-dodecylthio-2-acetoxypropyl | $C_{22}H_{46}NO_6PS$ | Calc. | 54.67 | 9.52 | 2.90 | 6.41 |
| 2-trimethyl-ammonioethyl phosphate | 483.27 | Found | 54.55 | 9.47 | 2.84 | 6.23 |
| 3-dodecylthio-2-propionyloxypropyl | $C_{23}H_{48}NO_6PS$ | Calc. | 55.55 | 9.65 | 2.82 | 6.23 |
| 2-trimethylammonioethyl phosphate | 497.28 | Found | 55.48 | 9.57 | 2.92 | 6.08 |

| | Formula Mol. Weight | | C | H | N | P |
|---|---|---|---|---|---|---|
| 3-hexadecylthio-2-propionyloxypropyl | $C_{27}H_{56}NO_6PS$ | Calc. | 58.60 | 10.12 | 2.53 | 5.60 |
| 2-trimethylammonioethyl phosphate | 553.32 | Found | 58.45 | 10.26 | 2.45 | 5.71 |
| 3-octadecylthio-2-acetoxypropyl | $C_{28}H_{58}NO_6PS$ | Calc. | 59.27 | 10.22 | 2.45 | 5.46 |
| 2-trimethylammonioethyl phosphate | 567.33 | Found | 59.05 | 10.02 | 2.53 | 5.42 |
| 3-octadecylthio-2-propionyloxypropyl | $C_{29}H_{60}NO_6PS$ | Calc. | 59.92 | 10.32 | 2.41 | 5.33 |
| 2-trimethylammonioethyl phosphate | 581.34 | Found | 59.71 | 10.17 | 2.31 | 5.40 |
| 3-eicosylthio-2-acetoxypropyl | $C_{30}H_{62}NO_6PS$ | Calc. | 60.52 | 10.41 | 2.35 | 5.20 |
| 2-trimethylammonioethyl phosphate | 595.35 | Found | 60.43 | 10.30 | 2.33 | 5.22 |
| 3-eicosyl-2-propionyloxypropyl | $C_{31}H_{64}NO_6PS$ | Calc. | 61.10 | 10.50 | 2.30 | 5.08 |
| 2-trimethylammonioethyl phosphate | 609.36 | Found | 61.01 | 10.42 | 2.32 | 5.02 |

**Claims for the Contracting States: BE, CH, FR, GB, IT, LI, LU, NL, SE**

1. 3-Hydrocarbylthio-2-acyloxypropyl 2-trimethylammonioethyl phosphates having the general formula I

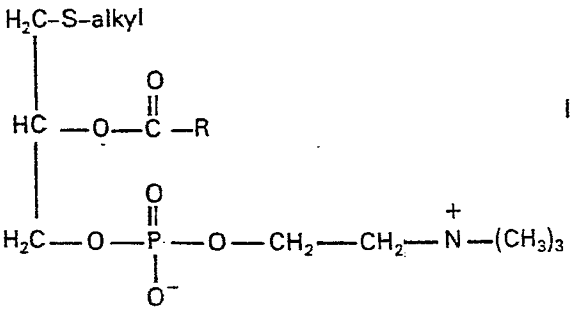

I

wherein alkyl is a saturated straight or branched acyclic hydrocarbon group with 6 to 20 carbon atoms or an unsaturated straight or branched acyclic hydrocarbon group with 6 to 20 carbon atoms containing one or two double bonds, and R represents methyl or ethyl.

2. 3-Hydrocarbylthio-2-acyloxypropyl 2-trimethylammonioethyl phosphates according to claim 1 characterized in that alkyl is a saturated straight or branched acyclic hydrocarbon group having from 12 to 20 carbon atoms or an unsaturated straight or branched acyclic hydrocarbon group having from 12 to 20 carbon atoms containing one or two double bonds.

3. 3-Hydrocarbylthio-2-acyloxypropyl 2-trimethylammonioethyl phosphates according to claim 1 or 2 characterized in that alkyl is a saturated straight chain acyclic hydrocarbon group with 12, 14, 16, 18 or 20 carbon atoms.

4. Process for the production of the 3-hydrocarbylthio-2-acyloxypropyl 2-trimethylammonioethyl phosphates according to claims 1 to 3 characterized in that 1-mercapto-2,3-propanediol is reacted with an alkyl-Hal, alkyl having the same meaning as in formula I and Hal representing a halogen atom, possibly in the presence of an alkaline condensation agent, blocking the primery hydroxy group in the 3-position of the resulting 1-hydrocarbylthio-2,3-propanediol by an appropriate protective group, esterifying the resulting protected product with a reactive derivative of a carboxylic acid of the formula R-CO-OH, R having the same meaning as in formula I, splitting off the protective group in the 3-position, reacting the resulting 1-hydrocarbylthio-2-O-acyl-2.3-propanediol of the general formula V

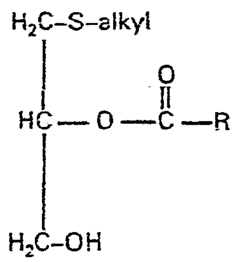

V

wherein alkyl and R have the same meaning as in formula I, with β-bromoethylphosphoric acid dichloride of the formula VI

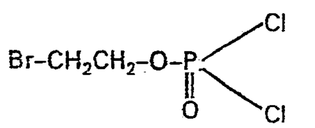

VI

and reacting the resulting 3-hydrocarbylthio-2-acyloxypropyl 2-bromoethyl hydrogen phosphate having the general formula VII

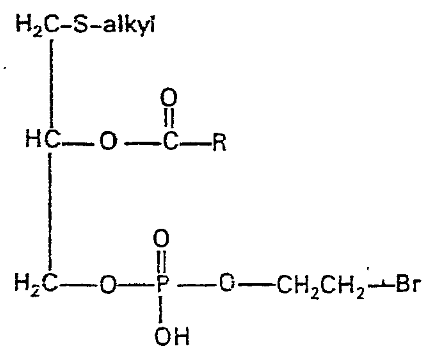

VII

wherein alkyl and R have the same meanings as in formula I, with trimethylamine and thereby converting it into the corresponding choline ester of formula I.

5. Pharmaceutical preparations comprising one or more of the compounds according to claim 1 to 3 as the active ingredient together with usual physiologically acceptable carriers and/or diluents.

**Claims for the Contracting State: AT**

1. Process for the production of 3-hydrocarbylthio-2-acyloxypropyl 2-trimethylammonioethyl phosphate having the general formula I

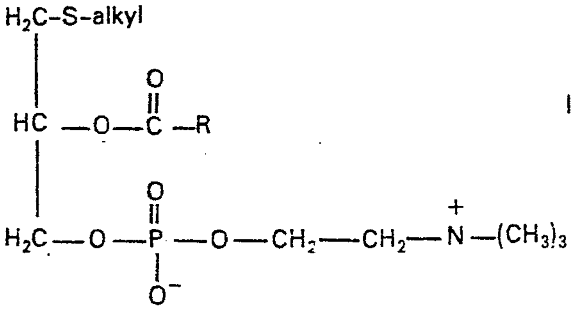

I

wherein Alkyl is a saturated straight or branched acyclic hydrocarbon group having from 6 to 20 carbon atoms or an unsaturated straight or branched acyclic hydrocarbon group with 6 to 20 carbon atoms containing one or two double bonds and R is the methyl or ethyl group characterized in that in manner known per se 1-mercapto-2,3-propanediol is reacted with an alkyl halide having the formula Alkyl-Hal, Alkyl having the same meaning as in formula I and Hal is a halogen atom, in particular a bromine or chlorine atom, possibly in the presence of an alkaline condensation agent, blocking the primery alcohol group in the 3-position of the resulting 1-hydrocarbylthio-2,3-propanediol with an appropriate protective group, esterifying the resulting protected compound with a reactive derivative of a carboxylic acid of the formula R-CO-OH, wherein R has the same meaning as in formula I, splitting of the protective group in the 3-position and reacting the resulting 1-hydrocarbylthio-2-O-acyl-2.3-propanediol of the general formula V

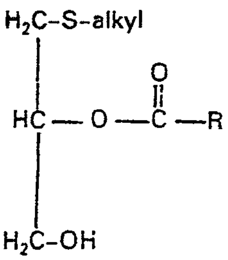

V

wherein Alkyl and R have the same meaning as in formula I, with β-bromo-ethyl-phosphoric acid dichloride of the general formula VI

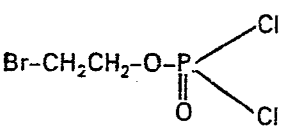

VI

and reacting the resulting 3-hydrocarbylthio-2-acyloxypropyl 2-bromoethyl hydrogen phosphate having the general formula VII

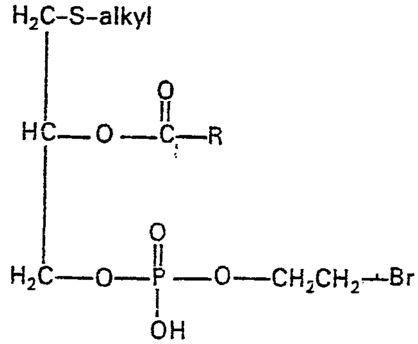

VII

wherein Alkyl and R have the same meaning as in formula I, with trimethylamine, thus converting it to the corresponding choline ester of formula I.

2. Process according to claim 1 characterized in that Alkyl in the 1-hydrocarbylthio-2-O-acyl-2,3-propanediols of general formula V is a saturated straight or branched acyclic hydrocarbon group having from 12 to 20 carbon atoms or an unsaturated straight or branched acyclic hydrocarbon group having from 12 to 20 carbon atoms containing one or two double bonds and R has the same meaning as in formula I.

3. Process according to claim 1 or 2, characterized in that Alkyl in the 1-hydrocarbylthio-2-O-acyl-2.3-propanediols of the general formula V is a saturated straight chain acyclic hydrocarbon group having 12, 14, 16, 18 or 20 carbon atoms and R has the same meaning as in formula I.

**Revendications pour les Etats contractants: BE, CH, FR, GB, IT, LI, LU, NL, SE**

1. 3-hydrocarbylthio-2-acyloxypropyl-2-triméthylammonioéthyl-phosphates de formule générale I

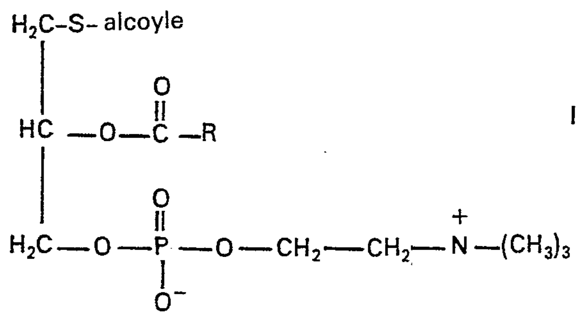

I

où alcoyle est un groupe hydrocarboné acyclique à chaîne droite ou ramifiée en $C_6$ à $C_{20}$ ou un groupe hydrocarboné acyclique à chaîne droite ou ramifiée insaturée en $C_6$ à $C_{20}$ contenant une ou deux doubles liaisons, et R représente un méthyle ou un éthyle.

2. 3-hydrocarbylthio-2-acyloxypropyl-2-triméthylammonioéthyl-phosphates selon la revendication 1, caractérisés en ce que l'alcoyle est un groupe hydrocarboné acyclique à chaîne droite ou ramifiée saturé en $C_{12}$ à $C_{20}$ ou un groupe hydrocarboné acyclique à chaîne droite ou ramifiée insaturé en $C_{12}$ à $C_{20}$ contenant 1 ou 2 doubles liaisons.

3. 3-hydrocarbylthio-2-acyloxypropyl-2-trimé-

thylammonioéthyl-phosphates selon l'une des revendications 1 ou 2, caractérisés en ce que l'alcoyle est un groupe hydrocarboné acyclique à chaîne droite saturée ayant 12, 14, 16, 18 ou 20 atomes de carbone.

4. Procédé de production des 3-hydrocarbylthio-2-acyloxypropyl-2-triméthylammonioéthylphosphates selon les revendications 1 à 3, caractérisé en ce qu'on fait réagir du 1-mercapto-2,3-propanediol avec un alcoyl-Hal, alcoyle ayant la même signification que dans la formule I et Hal représentant un atome d'halogène, éventuellement en présence d'un agent de condensation alcalin, en bloquant le groupe hydroxy primaire en position 3 du 1-hydrocarbylthio-2,3-propanediol obtenu par un groupe protecteur approprié, en estérifiant le produit protégé obtenu avec un dérivé réactif d'un acid carboxylique de formule R-CO-OH, R ayant la même signification que dans la formule I, en séparant le groupe protecteur en position 3, en faisant réagir le 1-hydrocarbylthio-2-O-acyl-2,3-propanediol obtenu de formule générale V

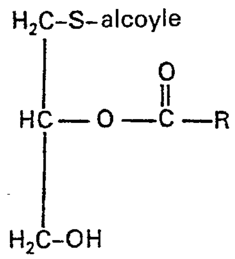

V

où alcoyle et R ont la même signification que dans la formule I, avec du dichlorure de l'acide β-bromoéthylphosphorique de formule VI

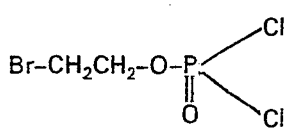

VI

et en faisant réagir le phosphate acide de 3-hydrocarbylthio-2-acyloxypropyl-2-bromoéthyle obtenu de formule générale VII

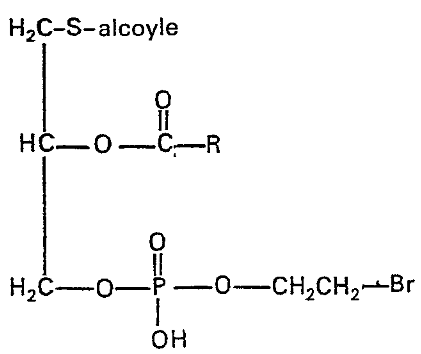

VII

où alcoyle et R ont la même signification que dans la formule I, avec de la triméthylamine et en le transformant ainsi en le choline-ester correspondant de formule I.

5. Préparations pharmaceutiques comprenant un ou plusieurs des composés selon l'une des revendications 1 à 3 comme ingrédient actif avec les supports et/ou diluants physiologiquement acceptables habituels.

**Revendications pour l'Etat contractant: AT**

1. Procédé de production de 3-hydrocarbylthio-2-acyloxypropyl-2-triméthylammonioéthylphosphate de formule générale I

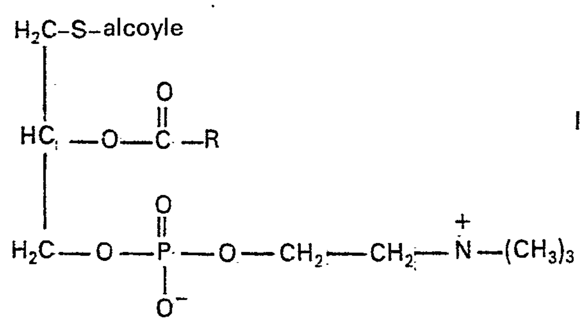

I

où alcoyle est un groupe hydrocarboné acyclique à chaîne droite ou ramifiée saturée en $C_6$ à $C_{20}$ ou un groupe hydrocarboné acyclique à chaîne droite ou ramifiée en $C_6$ à $C_{20}$ contenant 1 ou 2 doubles liaisons et R est le groupe méthyle ou éthyle, caractérisé en ce qu'on fait réagir de façon classique du 1-mercapto-2,3-propanediol avec un halogénure d'alcoyle de formule alcoyl-Hal, alcoyle ayant la même signification que dans la formule I et Hal étant un atome d'halogène, en particulier un atome de brome ou de chlore, éventuellement en présence d'un agent de condensation alcalin, en bloquant le groupe alcool primaire en position 3 du 1-hydrocarbylthio-2,3-propanediol obtenu avec un groupe protecteur approprié, en estérifiant le composé protégé obtenu avec un dérivé réactif d'un acid carboxylique de formule R-CO-OH, où R a la même signification que dans la formule I, en séparant le groupe protecteur en position 3 et en faisant réagir le 1-hydrocarbylthio-2-O-acyl-2,3-propanediol obtenu de formule générale

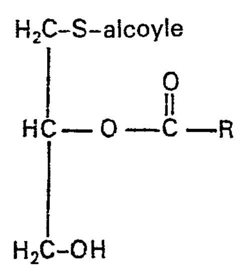

V

où alcoyle et R ont la même signification que dans la formule I, avec un dichlorure d'acide β-bromo-éthyl-phosphorique de formule générale VI

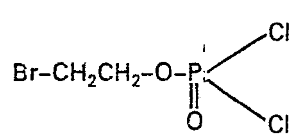

VI

et en faisant réagir le phosphate acide de 3-hydrocarbylthio-2-acyloxypropyl-2-bromoéthyle obtenu de formule générale VII

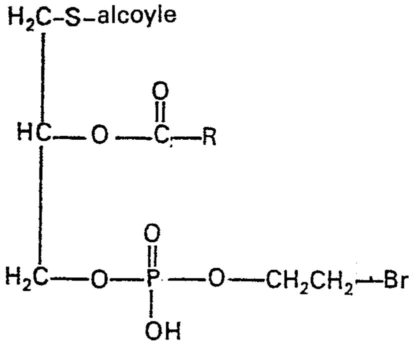

VII

où alcoyle et R ont la même signification que dans la formule I, avec de la triméthylamine, le transformant ainsi en le cholinester correspondant de formule I.

2. Procédé selon la revendication 1 caractérisé en ce que l'alcoyle dans les 1-hydrocarbylthio-2-O-acyl-2,3-propanediols de formule générale V est un groupe hydrocarboné acyclique à chaîne droite ou ramifiée saturé en $C_{12}$ à $C_{20}$ ou un groupe hydrocarboné acyclique à chaîne droite ou ramifiée insaturé en $C_{12}$ à $C_{20}$ contenant 1 ou 2 doubles liaisons et R a la même signification que dans la formule I.

3. Procédé selon l'une des revendications 1 ou 2, caractérisé en ce que l'alcoyle dans les 1-hydrocarbylthio-2-O-acyl-2,3-propanediols de formule générale V est un groupe hydrocarboné acyclique à chaîne droite saturé ayant 12, 14, 16, 18 ou 20 atomes de carbone et R a la même signification que dans la formule I.

**Patentansprüche für die Vertragsstaaten: BE, CH, FR, GB, IT, LI, LU, NL, SE**

1. 3-Hydrocarbylthio-2-acyloxypropyl-2-trimethylammoniumethylphosphat der allgemeinen Formel I

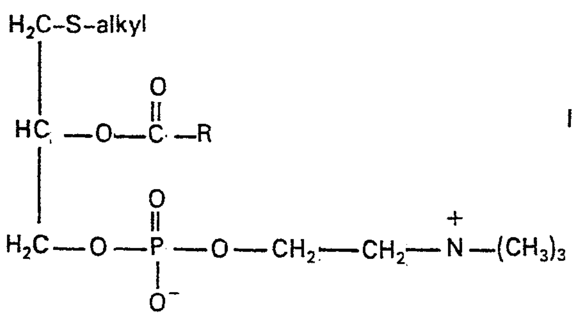

I

worin Alkyl eine gesättigte geradkettige oder verzweigte Kohlenwasserstoffgruppe mit 6 bis 20 Kohlenstoffatomen oder eine ungesättigte geradkettige oder verzweigte Kohlenwasserstoffgruppe mit 6 bis 20 Kohlenstoffatomen und einer oder zwei Doppelbindungen ist, und R Methyl oder Ethyl ist.

2. 3-Hydrocarbylthio-2-acyloxypropyl-2-trimethylammoniumethylphosphate gemäss Anspruch 1, dadurch gekennzeichnet, dass Alkyl eine gesättigte geradkettige oder verzweigte acyclische Kohlenwasserstoffgruppe mit 12 bis 20 Atomen oder ungesättigte, geradkettige oder verzweigte acyclische Kohlenwasserstoffgruppe mit 12 bis 20 Kohlenstoffatomen und 1 oder 2 Doppelbindungen ist.

3. 3-Hydrocarbylthio-2-acyloxypropyl-2-trimethylammoniumethylphosphate gemäss Anspruch 1 oder 2, dadurch gekennzeichnet, dass Alkyl eine gesättigte Kohlenwasserstoffgruppe mit 12, 14, 16, 18 oder 20 Kohlenstoffatomen ist.

4. Verfahren zur Herstellung der 3-hydrocarbylthio -2-acyloxypropyl-2-trimethylammoniumethylphosphate gemäss Anspruch 1 bis 3, dadurch gekennzeichnet, dass 1-Mercapto-2,3-propandiol, gegebenenfalls in Anwesenheit eines alkalischen Kondensationsmittels, mit einem Alkyl-Hal umgesetzt wird, wobei Alkyl die gleiche Bedeutung wie in Formel I hat und Hal ein Halogenatom ist, die primare Hydroxygruppe in der 3-Stellung des erhaltenen 1-Hydrocarbylthio-2,3-propandiols durch eine geeignete Schutzgruppe blockiert wird, das erhaltene geschützte Produkt mit einem reaktionsfähigen Derivat einer Carbonsäure der Formel R-COOH, worin R die gleiche Bedeutung wie in Formel I hat, umgesetzt wird, die Schutzgruppe in der 3-Stellung abgespalten wird, das erhaltene Umsetzungsprodukt 1-Hydrocarbylthio-2-O-acyl-2,3-propandiol der allgemeinen Formel V

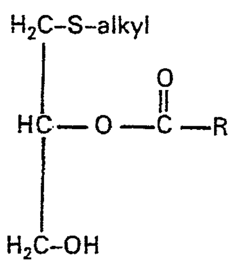

V

worin Alkyl und R die gleiche Bedeutung wie in Formel I haben, mit β-Bromethylphosphorsäuredichlorid der Formel VI

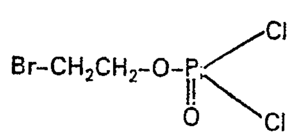

VI

und das erhaltene 3-Hydrocarbylthio-2-acyloxydpropyl-2-bromethylhydrogenphosphat der allgemeinen Formel VII

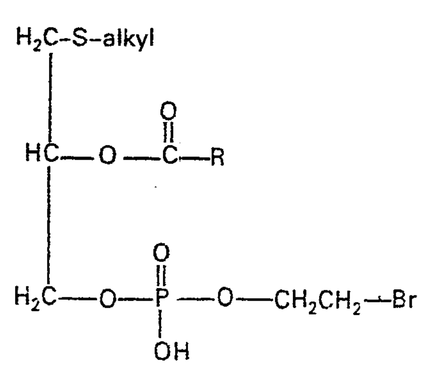

VII

worin Alky und R die gleiche Bedeutung wie in Formel I haben, mit Trimethylamin zum entsprechenden Cholinester der Formel I umgesetzt wird.

5. Pharmazeutische Produkte enthaltend eine oder mehrere Verbindungen gemäss Anspruch 1 bis 3 als Wirkstoff zusammen mit üblichen physiologisch verträglichen Trägermaterialien und/oder Verbindungsmitteln ist.

**Patentansprüche für den Vertragsstaat: AT**

1. Verfahren zur Herstellung von 3-Hydrocarbylthio-2-acyloxypropyl-2-trimethylammoniumethylphosphat der allgemeinen Formel I

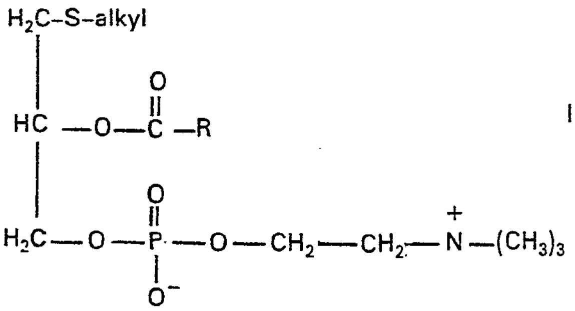

I

worin Alkyl eine gesättigte geradkettige oder verzweigte Kohlenwasserstoffgruppe mit 6 bis 20 Kohlenstoffatomen oder eine ungesättigte geradkettige oder verzweigte Kohlenwasserstoffgruppe mit 6 bis 20 Kohlenstoffatomen und einer oder zwei Doppelbindungen ist, und R Methyl oder Ethyl ist, dadurch gekennzeichnet, dass 1-Mercapto-2,3-propandiol, gegebenenfalls in Anwesenheit eines alkalischen Kondensationsmittels, mit einem Alkyl-Hal umgesetzt wird, wobei Alkyl die gleiche Bedeutung wie in Formel I hat und Hal ein Halogenatom ist, die primäre Hydroxygruppe in der 3-Stellung des erhaltenen 1-Hydrocarbylthio-2,3-propandiols durch eine geeignete Schutzgruppe blockiert wird, das erhaltene geschützte Produkt mit einem reaktionsfähigen Derivat einer Carbonsäure der Formel R–COOH, worin R die gleiche Bedeutung wie in Formel I hat, umgesetzt wird, die Schutzgruppe in der 3-Stellung abgespalten wird, das erhaltene Umsetzungsprodukt 1-Hydrocarbylthio-2-O-acyl-2,3-propandiol der allgemeinen Formel V

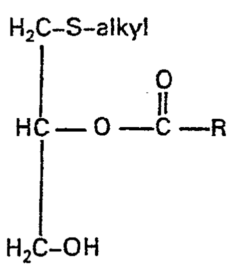

V

worin Alkyl und R die gleiche Bedeutung wie in Formel I haben, mit β-Bromethylphosphorsäuredichlorid der Formel VI

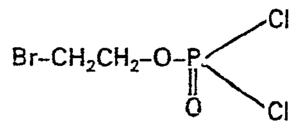

VI

und das erhaltene 3-Hydrocarbylthio-2-acyloxydpropyl-2-bromethylhydrogenphosphat der allgemeinen Formel VII

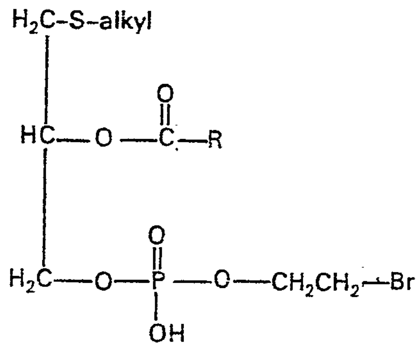

VII

worin Alkyl und R die gleiche Bedeutung wie in Formel I haben, mit Trimethylamin zum entsprechenden Cholinester der Formel I umgesetzt wird.

2. Verfahren gemäss Anspruch 1, dadurch gekennzeichnet, dass Alkyl 1-Hydrocarbylthio-2-O-acyl-2,3-propandiols der allgemeinen Formel V eine gesättigte, geradkettige oder verzweigte, acyclische Kohlenwasserstoffgruppe mit 12 bis 20 Kohlenwasserstoffatomen oder eine ungesättigte, geradkettige oder verzweigte acyclische Kohlenwasserstoffgruppe mit 12 bis 20 Kohlenwasserstoffatomen und einer oder zwei Doppelbindungen ist und R die gleiche Bedeutung wie in Formel I hat.

3. Verfahren gemäss Anspruch 1 oder 2, dadurch gekennzeichnet, dass Alkyl in den 1-Hydrocarbylthio-2-O-acyl-2,3-propandiols der allgemeinen Formel V eine gesättigte, geradkettige, acyclische Kohlenwasserstoffgruppe mit 12, 14, 16, 18 oder 20 Kohlenstoffatomen ist und R die gleiche Bedeutung wie in Formel I hat.